(19) Europäisches Patentamt
European Patent Office
Office européen des brevets

(11) Publication number : **0 285 284 B1**

# (12) EUROPEAN PATENT SPECIFICATION

(45) Date of publication of patent specification : **15.05.91 Bulletin 91/20**

(51) Int. Cl.$^5$ : **C07C 311/08**, C07C 237/30, A61K 31/165

(21) Application number : **88302270.9**

(22) Date of filing : **16.03.88**

## (54) N-phenethylaminoalkyl-benzamide anti-arrhythmia agents.

(30) Priority : **20.03.87 GB 8706662**

(43) Date of publication of application : **05.10.88 Bulletin 88/40**

(45) Publication of the grant of the patent : **15.05.91 Bulletin 91/20**

(84) Designated Contracting States : **AT BE CH DE ES FR GB GR IT LI LU NL SE**

(56) References cited :
**EP-A- 0 158 775**
**EP-A- 0 164 865**
**EP-A- 0 167 245**
**EP-A- 0 178 874**

(73) Proprietor : **Pfizer Limited**
**Ramsgate Road**
**Sandwich Kent CT13 9NJ (GB)**

(72) Inventor : **Cross, Peter Edward, Dr.**
**21 Cherry Avenue**
**Canterbury Kent (GB)**
Inventor : **Arrowsmith, John Edmund, Dr.**
**340 Dover Road, Upper Walmer**
**Deal Kent (GB)**

(74) Representative : **Moore, James William, Dr.**
**Pfizer Limited Ramsgate Road**
**Sandwich Kent CT13 9NJ (GB)**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Jouve, 18, rue Saint-Denis, 75001 PARIS

## Description

This invention relates to certain N-phenethylaminoalkyl-benzamide derivatives which are anti-arrhythmia agents useful for the treatment of cardiac conditions.

The compounds of the invention prolong the duration of the action potential in cardiac muscle and conducting tissue, and thereby increase refractoriness to premature stimuli. Thus, they are Class III anti-arrhythmic agents according to the classification of Vaughan Williams (Anti-Arrhythmic Action, E. M. Vaughan Williams, Academic Press, 1980). They are effective in atria, ventricles and conducting tissue both in vitro and in vivo and are therefore useful for the prevention and treatment of a wide variety of ventricular and supraventricular arrhythmias including atrial and ventricular fibrillation. Because they do not alter the speed at which impulses are conducted, they have less propensity than current drugs (mostly Class I) to precipitate or aggravate arrhythmias, and they also produce less neurological side effects. Some of the compounds also have positive inotropic activity and therefore are particularly beneficial in patients with impaired cardiac pump function.

Thus the invention provides compounds of the formula :

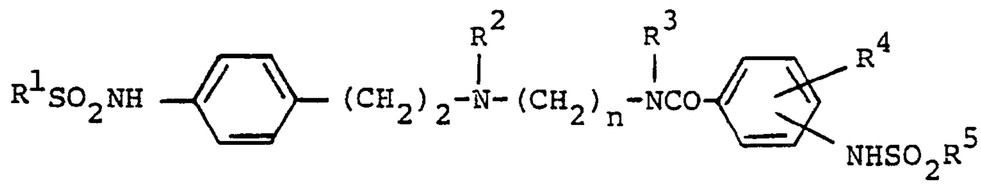

--- (I)

and their pharmaceutically acceptable salts,

wherein $R^1$ and $R^5$ are each independently $C_1$-$C_4$ alkyl ;

$R^2$ and $R^3$ are each independently H or $C_1$-$C_4$ alkyl ;

$R^4$ is H or $C_1$-$C_4$ alkoxy,

and n is 2, 3 or 4.

The pharmaceutically acceptable salts of the compounds of the formula (I) include acid addition salts formed from acids which form non-toxic salts such as the hydrochloride, hydrobromide, hydroiodide, sulphate or bisulphate, phosphate or hydrogen phosphate, acetate, maleate, fumarate, lactate, tartrate, citrate, gluconate, benzoate, methanesulphonate, benzenesulphonate and p-toluenesulphonate salts. Some of the compounds may also form metal salts, particularly alkaline earth and alkali metal salts. Examples include the sodium and potassium salts.

$R^1$ and $R^5$ are preferably both $CH_3$, $R^2$ is preferably $CH_3$ or $CH_2CH_3$, $R^3$ is preferably H, $R^4$ is preferably H or $OCH_3$ and n is preferably 2.

Thus in one particular and preferred aspect, the invention provides the compound of formula (I) wherein $R^1$ is $CH_3$, $R^2$ is $CH_3$, $R^3$ is H, $R^4$ is methoxy attached at the 2-position of the phenyl ring and $R^5$ is $CH_3$, the group $-NHSO_2R^5$ being attached at the 4-position of the phenyl ring, and n is 2.

The invention also provides novel compounds of the following formula :

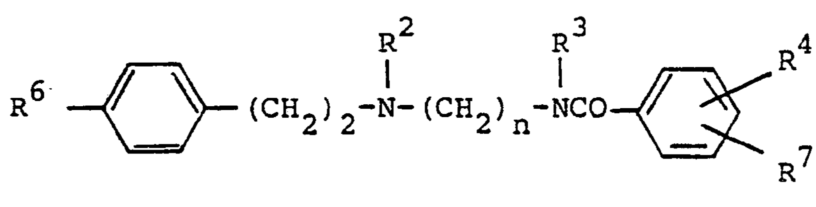

--- (II)

wherein $R^2$, $R^3$, $R^4$ and n are as previously defined and $R^6$ and $R^7$ are each $NH_2$ or $NO_2$, or $R^6$ is ($C_1$-$C_4$ alkyl)$SO_2NH$ and $R^7$ is $NH_2$ or $NO_2$.

These compounds are valuable intermediates for the preparation of the antiarrhythmic compounds of formula (I).

The compounds of formula (I) are prepared from a compound of the formula :

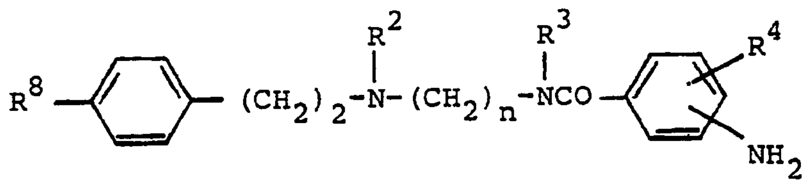

--- (III)

wherein $R^2$, $R^3$, $R^4$ and n are as previously defined and $R^8$ is $NH_2$ or ($C_1$-$C_4$ alkyl)$SO_2NH$–, by reacting with a $C_1$-$C_4$ alkyl sulphonylchloride or bromide, or a sulphonic anhydride of formula [($C_1$-$C_4$ alkyl)$SO_2$]$_2$O.

The reaction is typically carried out by stirring the reactants together in an organic solvent at room temperature for several hours. When a sulphonyl halide is used, the reaction is generally performed in pyridine but with a sulphonic anhydride, methylene chloride in a more suitable solvent. The product of formula (I) is isolated by removal of the solvent and purified by conventional procedures such as crystallisation or chromatography. In the case where $R^8$ is $NH_2$, $R^1$ and $R^5$ in the product of formula (I) will naturally be the same depending on the particular reagent used.

The starting materials of formula (III) wherein $R^8$ is $NH_2$ are prepared from readily available starting materials as shown in the following reaction scheme wherein $R^2$, $R^3$, $R^4$ and n are as previously defined and Q is a good leaving group, for example Cl, Br, I, methanesulphonyloxy, phenylsulphonyloxy or para-toluenesulphonyloxy ; bromine being generally preferred :

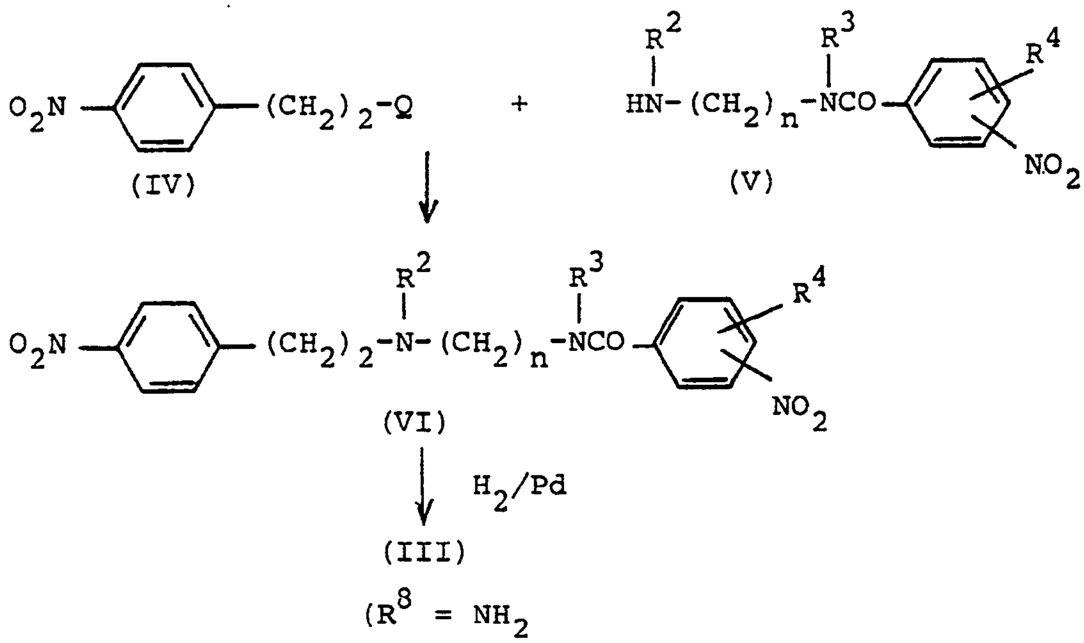


(IV) (V)

(VI)

(III)

($R^8$ = $NH_2$

The initial reaction between the compounds of formula (IV) and (V) is typically achieved by heating the reactants in an organic solvent in the presence of an acid binding agent such as potassium carbonate. For example the reaction may be achieved by refluxing the reactants in acetonitrile for a period of one or two days. The product (VI) is then reduced, for example by catalytic hydrogenation over palladium on charcoal to yield the amine starting material of formula (III) wherein $R^8$ is $NH_2$.

The starting compounds of formula (III) wherein $R^8$ is ($C_1$-$C_4$ alkyl)$SO_2NH$ are prepared as shown in the following reaction scheme wherein X is halogen, preferably chlorine and Q is a leaving group as previously defined :

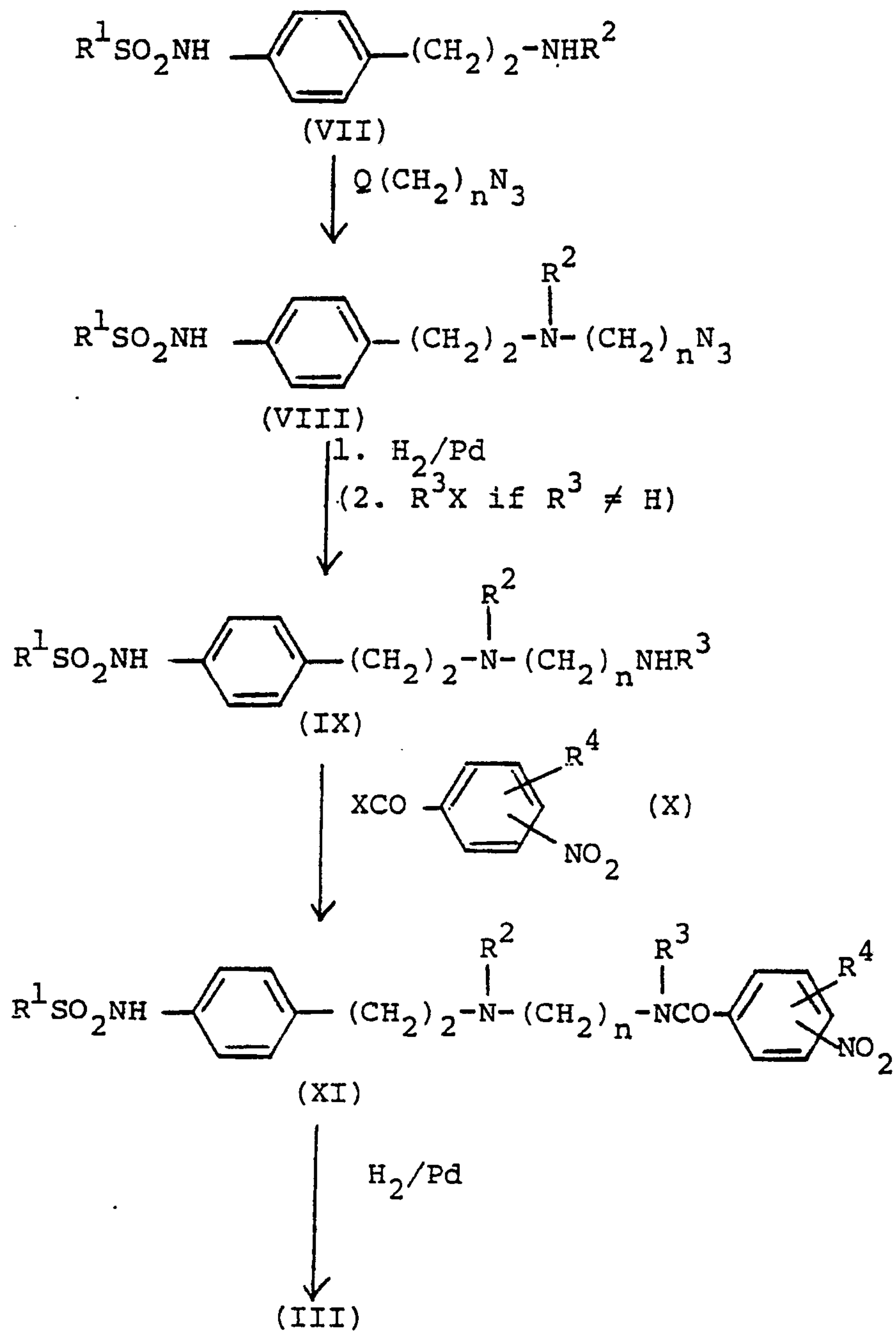


(VII)

(VIII)

(IX)

(X)

(XI)

(III)

$R^8 = R^1SO_2NH$

The process involves reacting the amine of formula (VII) with, for example, an azido-O-methanesulphonyl alkanol to yield the azide (VIII) which is reduced by catalytic hydrogenation to give the amine (IX) wherein $R^3$ is hydrogen. If desired, this product can be alkylated, for example by reaction with an alkyl halide, to give compounds of formula (IX) wherein $R^3$ is $C_1$-$C_4$ alkyl.

In the next step, the reaction between the amine (IX) and the benzoyl halide (X) is typically carried out by stirring the reactants in equimolar proportions in an organic solvent, for example butan-2-one, either at room temperature or for a shorter period under reflux. The product (XI) is finally reduced as previously described to yield the starting material of formula (III) wherein $R^8$ is ($C_1$-$C_4$ alkyl)$SO_2NH$. This route will of course be used when it is desired that $R^1$ and $R^5$ in the product of formula (I) should be different.

All of the above reactions are conventional and appropriate reagents and reaction conditions for their performance and procedures for isolating the desired products will be well known to those skilled in the art in accordance with literature precedents and by reference to the Examples hereto.

Pharmaceutically acceptable salts are readily prepared by mixing solutions containing equimolar amounts of the free base and the desired acid. The salt generally precipitates from solution or is recovered by evaporation of the solvent.

The biological activity of the compounds of the invention is assessed by measuring the effect of the compounds on atrial refractoriness. In this test guinea pig right hemiatria are mounted in a bath containing physiological salt solution, with one end connected to a force transducer. The tissues are stimulated at 1 Hz using field electrodes. Effective refractory period (ERP) is measured by introducing premature stimuli ($S_2$) after every 8th basic stimulus ($S_1$). The $S_1S_2$ coupling interval is gradually increased until $S_2$ reproducibly elicits a propagated response. This is defined as the ERP. The test compound is then added to the bath and the concentration of compound required to increase ERP by 25% is determined ($ED_{25}$). ERP is also measured in guinea pig right papillary muscles incubated in physiological saline solution. Muscles are stimulated at one end using bipolar electrodes and the propagated electrogram is recorded at the opposite end via a unipolar surface electrode. ERP is determined as above using the extrastimulus technique. Conduction time is obtained from a digital storage oscilloscope by measuring the interval between the stimulus artefact and the peak of the electrogram (i.e. the time required for the impulse to travel along the length of the muscle).

Atrial and vetricular ERP's are also measured in anaesthetised or conscious dogs by the extrastimulus technique whilst the atrium or right ventricle is being paced at a constant rate.

For human use the compounds of the formula (I) can be administered alone but will generally be administered in admixture with a pharmaceutical carrier selected with regard to the intended route of administration and standard pharmaceutical practice. They can be administered both to patients suffering from arrhythmias and also prophylactically to those likely to develop arrhythmias. For example they may be administered orally in the form of tablets containing such excipients as starch or lactose, or in capsules either alone or in admixture with excipients, or in the form of elixirs or suspensions containing flavouring or colouring agents. They may be injected parenterally, for example, intravenously, intramuscularly or subcutaneously. For parenteral administration, they are best used in the form of a sterile aqueous solution which may contain other solutes, for example, enough salts or glucose to make the solution isotonic with blood.

For administration to man in the curative or prophylactic treatment of cardiac conditions such as ventricular and supraventricular arrhythmias, including atrial and ventricular fibrillation, it is expected that oral dosages of the compounds of the invention will be in the range from 1 to 75 mg daily, taken in up to 4 divided doses per day, for an average adult patient (70 kg). Thus for a typical adult patient, individual tablets or capsules might contain 1 to 25 mg of active compound, in a suitable pharmaceutically acceptable vehicle or carrier. Dosages for intravenous administration would be expected to be within the range 0.5 to 10 mg per single dose as required. A severe cardiac arrhythmia is preferably treated by the i.v. route in order to effect a rapid conversion to the normal rhythm. Variations on these dosages may occur depending on the weight and condition of the subject being treated as will be determined by the medical practitioner.

Thus the present invention provides a pharmaceutical composition comprising a compound of the formula (I) as defined above or pharmaceutically acceptable salt thereof, together with a pharmaceutically acceptable diluent or carrier.

The invention also provides a method of preventing or reducing cardiac arrhythmias in a human being, which comprises administering to said human an effective amount of a compound of formula (I) or pharmaceutically acceptable salt thereof, or a pharmaceutical composition as defined above.

The invention yet further provides a compound of the formula (I) or pharmaceutically acceptable salt thereof, for use as an anti-arrhythmia agent.

The invention also provides the use of a compound of the formula (I), or a pharmaceutically acceptable salt thereof, for the manufacture of a medicament for the prevention or reduction of cardiac arrhythmias.

The preparation of the compounds of the invention, the intermediates of formula (II) and certain starting materials therefor are illustrated by the following Examples :

EXAMPLE 1

N-2-(Methylamino)ethyl-4-nitrobenzamide

4-Nitrobenzoyl chloride (8.2 g, 44 mmole) was added portionwise with simultaneous dropwise addition of 48% hydrobromic acid (11 ml, 135 mmole) to a stirred solution of N-methylethylenediamine (10 g, 135 mmole) in a mixture of ethanol (70 ml) and water (10 ml), maintaining the temperature below 40°C. The mixture was

heated at reflux temperature for 1 hour, cooled, and stored at 5°C overnight. The precipitate was removed by filtration and the filtrate was evaporated to dryness. The residue was taken up in water, the acidity adjusted to pH 1.5 with hydrochloric acid and the solution chilled and filtered. The filtrate was basified with sodium hydroxide solution and extracted with methylene chloride (5 × 25 ml). The organic layers were combined, dried over sodium sulphate and evaporated. The residue was recrystallised from ethyl acetate to give the title compound (2.9 g 29%). m.p. 120-121°C. Found : C,53.8 ; H,5.9 ; N,18.7. $C_{10}H_{13}N_3O_3$ requires C,53.8 ; H,5.9 ; N,18.8%.

EXAMPLE 2

N-2-(Ethylamino)ethyl-4-nitrobenzamide

4-Nitrobenzoyl chloride (8.2 g, 44 mmole), 48% hydrobromic acid (11 ml, 135 mmole) and N-ethyl ethylenediamine (11.9 g, 135 mmole) were reacted following the procedure of Example 1 to give the title compound (3.0 g, 33%). m.p. 90-92°C. Found : C,55.5 ; H,6.4 ; N,17.6. $C_{11}H_{15}N_3O_3$ requires : C,55.7 ; H,6.4 ; N,17.7%.

EXAMPLE 3

N-[2-(N'-4-Nitrophenethyl-N'-methylamino)ethyl]-4-nitrobenzamide

N-2-(Methylamino)ethyl-4-nitrobenzamide (1.5 g, 6.7 mmole), 4-nitrophenethyl bromide (1.54 g, 6.7 mmole) and potassium carbonate (0.93 g, 6.7 mmole) in acetonitrile (50 ml) were stirred at reflux temperature for 2 days. The solvent was evaporated and the residue diluted with water and extracted with methylene chloride (3 × 20 ml). The organic layer was washed twice with aqueous sodium carbonate and three times with brine, dried over sodium sulphate and evaporated. The resulting oil was purified by column chromatography on silica eluting with methylene chloride containing methanol (0% up to 2% by volume). The appropriate fractions were combined and the solvent evaporated to give the title compound (1.6 g, 64%). Found : C,58.0 ; H,5.3 ; N,14.95. $C_{18}H_{20}N_4O_5$ requires C,58.1 ; H,5.4 ; N,15.05%.

EXAMPLE 4

N-[2-(N'-4-Nitrophenethyl-N'-ethylamino)ethyl]-4-nitrobenzamide

N-2-(Ethylamino)ethyl-4-nitrobenzamide (2 g, 8.4 mmole), 4-nitrophenethyl bromide (1.94 g, 8.4 mmole), and potassium carbonate (1.17 g, 8.4 mmole) were heated in acetonitrile following the procedure of Example 3 to give the title compound (0.90 g). Found : C,58.75 ; H,5.90 ; N,14.3. $C_{19}H_{22}N_4O_5$ requires C,59.1 ; H,5.7 ; N,14.30%.

EXAMPLE 5

N-[2-(N'-4-Aminophenethyl-N'-methylamino)ethyl]-4-aminobenzamide

N-[2-(N'-4-Nitrophenethyl-N'-methylamino)ethyl-4-nitrobenzamide (1.2 g, 3.2 mmole) was stirred in ethanol with 5% palladium on charcoal (0.15 g), under a hydrogen atmosphere (3.45 bar) for 4 hours. The reaction mixture was filtered and the filtrate evaporated to dryness to give an oil, which was triturated with ethyl acetate and evaporated to give the title compound as a foam, (1.0 g, 99%) which was used without further purification. Found : C,67.9 ; H,7.9 ; N,17.0. $C_{18}H_{20}N_4O_5.0.25$ $(CH_3CO_2C_2H_5)$ requires C,68.2 ; H,7.8 ; N,16.75%.

EXAMPLE 6

N-[2-(N'-4-Aminophenethyl-N'-ethylamino)ethyl]-4-aminobenzamide

N-[2-(N'-4-Nitrophenethyl-N'-ethylamino)ethyl-4-nitrobenzamide (0.85 g, 2.2 mmole) was hydrogenated following the procedure of Example 5 to give the title compound (0.48 g). $^1$H-n.m.r. ($CDCl_3$) δ = 7.10 (q, 4H) ; 6.90 (q, 4H) ; 3.45 (q, 4H), 2.70 (m, 8H) ; 1.05 (t, 3H).

EXAMPLE 7

N-[2-(N′-4-Methanesulphonamidophenethyl-N′-methylamino)ethyl]-4-methanesulphonamidobenzamide, hemihydrate

Methanesulphonyl chloride (0.55 ml, 7.0 mmole) was added dropwise to a stirred solution of N-[2-(N′-4-aminophenethyl-N′-methylamino)ethyl]-4-aminobenzamide (0.9 g, 3.1 mmole) in pyridine (40 ml) at 0°C and the mixture stirred at room temperature for 18 hours. The solvent was removed by evaporation and the residue suspended in sodium bicarbonate solution which was extracted with methylene chloride (3 × 20 ml). The combined organic layers were washed twice with sodium bicarbonate solution and three times with brine, dried over sodium sulphate and evaporated to give an oil which was chromatographed on silica eluting with methylene chloride containing methanol (0% up to 5% by volume). The appropriate fractions were combined and evaporated to give the title compound as a colourless foam (0.60 g 43%). Found : C,50.65 ; H,6.1 ; N,11.6. $C_{20}H_{28}N_4O_5S_2.\frac{1}{2}H_2O$ requires C,50.3 ; H,6.1 ; N,11.7%.

EXAMPLE 8

N-[2-(N′-4-Methanesulphonamidophenethyl-N′-ethylamino)ethyl]-4-methanesulphonamidobenzamide

N-[2-(N′-4-Aminophenethyl-N′-ethylamino)ethyl]-4-aminobenzamide (0.48 g, 1.47 mmole) was reacted with methanesulphonyl chloride (0.23 ml, 3.0 mmole) following the procedure of Example 7 to give the title compound (0.26 g). Found : C,51.9 ; H,6.4 ; H,11.4. $C_{21}H_{23}N_4O_5S_2$ requires C,52.3 ; H,6.3 ; N,11.6%.

EXAMPLE 9

N-[2-(N′-4-Methanesulphonamidophenethyl-N′-methylamino)ethyl]-2-methoxy-4-nitrobenzamide hemihydrate

1. 2-Azido-O-methanesulphonyl-ethanol

Methanesulphonyl chloride (5.7 g, 5 mmole) in methylene chloride solution was added dropwise over $\frac{1}{2}$hour to a stirred solution of 2-azidoethanol (4.3 g, 50 mmole) and triethylamine (5.0 g, 50 mmole) in methylene chloride (80 ml). After 2 hours the reaction mixture was washed with water, dried over magnesium sulphate and evaporated to dryness to give the title compound as a mobile oil (7.0 g).

2. 2-Azido-N-(4-methanesulphonamidophenethyl)-N-methyl-ethylamine

A mixture of N-(4-Methanesulphonamidophenethyl)methylamine (6.1 g, 26.8 mmole), 2-azido-O-methane sulphonyl-ethanol (4.4 g, 26.7 mmole) and sodium bicarbonate (5 g) in acetonitrile (100 ml) was heated at reflux temperature for 2.5 hours. The solvent was removed and the residue extracted with methylene chloride. The extract was filtered and the solvent evaporated. The residue was chromatographed on silica, eluting with methylene chloride containing methanol (from 0% up to 2% by volume). The appropriate fractions were combined and evaporated to give the title azide as an oil (5.4 g, 68%).

3. N-(4-Methanesulphonamidophenethyl)-N-methylethylenediamine

The above product (0.9 g, 33 mmole) in ethanol (50 ml) was stirred under an atmosphere of hydrogen (3.45 bar) at room temperature for 2 hours in the presence of 5% palladium on charcoal catalyst (0.1 g). The reaction mixture was filtered and evaporated to give the title diamine as a powder (0.51 g, 62%), m.p. 90-92°C. Found: C,52.8 ; H,7.8 ; N,15.2. $C_{12}H_{21}N_3O_2S$ requires C,53.1 ; H,7.8 ; N,15.5%.

4.N-[2-(N′-4-Methanesulphonamidophenethyl-N′-methylamino)ethyl]-2-methoxy-4-nitrobenzamide hemihydrate

N-(4-Methanesulphonamidophenethyl)-N-methylethylenediamine (0.48 g, 1.9 mmole) and 2-methoxy-4-nitrobenzoyl chloride (0.4 g, 1.9 mmole) in butan-2-one were stirred at room temeprature for 4 hours and then heated at reflux temperature for 1 hour. The solvent was removed under reduced pressure and the residue diluted with aqueous sodium bicarbonate solution and extracted with methylene chloride (3 × 30 ml). The combined organic layers were washed three times with brine, dried over sodium sulphate and evaporated to an oil

which was chromatographed on silica eluting with methylene chloride containing methanol (0% up to 2% by volume). The appropriate fractions were combined and evaporated to give the title compound as a foam, (0.51 g, 64%). Found : C,52.0 ; H,5.8 ; N,11.8. $C_{20}H_{26}N_4O_6S.\frac{1}{2}H_2O$ requires C,52.3 ; H,5.9 ; H,12.2%.

EXAMPLE 10

N-[2-(N′-4-Methanesulphonamidophenethyl-N′-methylamino)ethyl]-4-amino-2-methoxybenzamide

N-[2-(N′-4-Methanesulphonamidophenethyl-N′-methylamino)ethyl]-2-methoxy-4-nitrobenzamide (0.5 g, 1.19 mmole) was converted to the title compound by catalytic hydrogenation following the procedure of Example 5. Yield 0.45 g. $^1$H-n.m.r. ($CDCl_3$) $\delta$ = 8.00 (d, 1H) ; 7.15 (q, 4H) ; 6.35 (q, 1H) ; 6.25 (d, 1H) ; 4.00 (s, 2H) ; 3.85 (s, 3H) ; 3.50 (q, 2H) ; 2.95 (s, 3H) ; 2.75 (d, 2H) ; 2.70 (d, 2H) ; 2.65 (q, 2H) ; 2.40 (s, 3H).

EXAMPLE 11

N-[2-(N′-4-Methanesulphonamidophenethyl-N′-methylamino)ethyl]-4-methanesulphonamido-2-methoxybenzamide

N-[2-(N′-4-Methanesulphonamidophenethyl-N′-methylamino)ethyl]-4-amino-2-methoxybenzamide (0.44 g, 1.0 mmole) was reacted with methanesulphonyl chloride (0.081 ml, 1.0 mmole) following the procedure of Example 7 to yield the title product (0.23 g, 47%). Found : C,50.5 ; H,6.05 ; N,11.0. $C_{21}H_{30}N_4O_6S_2$ requires C,50.8 ; H,6.1 ; N,11.2%.

## Claims

**C laims for the Contracting States : AT, BE, CH, DE, FR, GB, IT, LI, LU, NL, SE**

1. A compound having the formula :

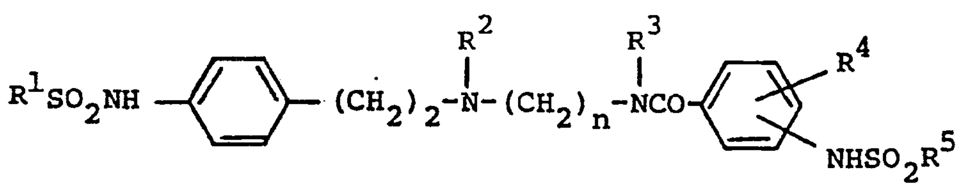

--- (I)

or a pharmaceutically acceptable salt thereof,
wherein $R^1$ and $R^5$ are each independently $C_1$-$C_4$ alkyl ;

$R^2$ and $R^3$ are each independently H or $C_1$-$C_4$ alkyl ;

$R^4$ is H or $C_1$-$C_4$ alkoxy,
and n is 2, 3 or 4.

2. A compound as claimed in claim 1 wherein $R^1$ and $R^5$ are both $CH_3$.
3. A compound as claimed in claim 1 or claim 2 wherein $R^2$ is $CH_3$ or $CH_2CH_3$ and $R^3$ is H.
4. A compound as claimed in any one of claims 1 to 3 wherein $R^4$ is H or $OCH_3$.
5. A compound as claimed in any one of claims 1 to 4 wherein n is 2.
6. The compound of formula (I) wherein $R^1$ is $CH_3$, $R^2$ is $CH_3$, $R^3$ is H, $R^4$ is $CH_3O$ attached at the 2-position of the phenyl ring and $R^5$ is $CH_3$, the group $-NHSO_2R^5$ being attached at the 4-position of the phenyl ring, and n is 2.
7. A pharmaceutical composition comprising a compound of the formula (I) or a pharmaceutically acceptable salt thereof as claimed in any one of claims 1 to 6 together with a pharmaceutically acceptable diluent or carrier.
8. A compound of the formula (I) or a pharmaceutically acceptable salt thereof as claimed in any one of claims 1 to 6 for use in medicine, in particular for use as an anti-arrhythmia agent.
9. The use of a compound of the formula (I) or a pharmaceutically acceptable salt thereof as claimed in

any one of claims 1 to 6, for the manufacture of a medicament for the prevention or reduction of cardiac arrhythmias.

10. A compound of the formula :

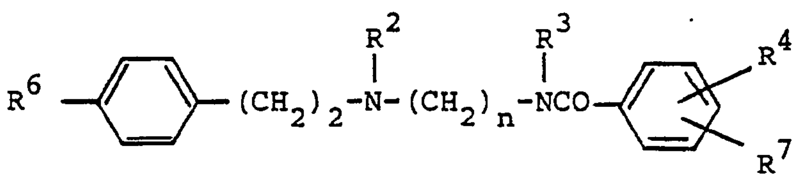

--- (II)

wherein $R^2$, $R^3$, $R^4$ and n are as defined in claim 1 and $R^6$ and $R^7$ are each $NH_2$ or $NO_2$, or $R^6$ is ($C_1$-$C_4$ alkyl)$SO_2NH$ and $R^7$ is $NH_2$ or $NO_2$.

**Claims for the following Contracting State : ES**

1. A process for preparing a compound having the formula :

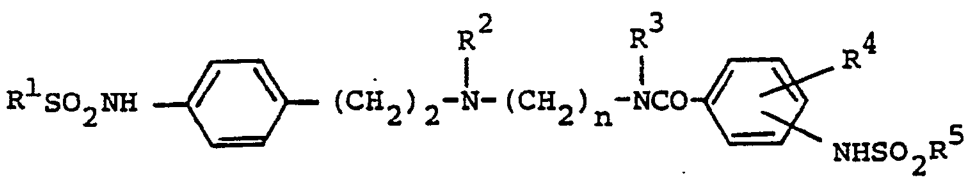

--- (I)

or a pharmaceutically acceptable salt thereof,

wherein $R^1$ and $R^5$ are each independently $C_1$-$C_4$ alkyl,

$R^2$ and $R^3$ are each independently H or $C_1$-$C_4$ alkyl ;

$R^4$ is H or $C_1$-$C_4$ alkoxy,

and n is 2, 3 or 4, which comprises reacting a compound of the formula :

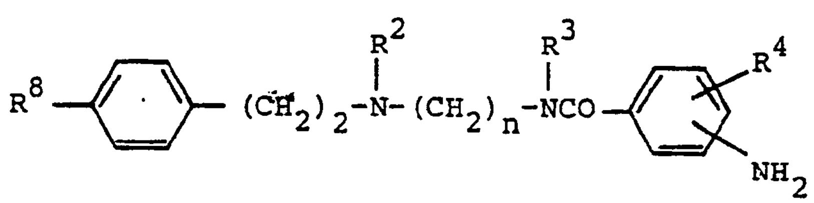

--- (III)

wherein $R^2$, $R^3$, $R^4$ and n are as previously defined and $R^8$ is $NH_2$ or ($C_1$-$C_4$ alkyl)$SO_2NH$ ; with a $C_1$-$C_4$ alkyl sulphonylchloride or bromide, or a sulphonic anhydride of formula $[(C_1\text{-}C_4\ \text{alkyl})SO_2]_2O$.

2. A process as claimed in claim 1 wherein $R^1$ and $R^5$ are both $CH_3$.

3. A process as claimed in claim 1 or claim 2 wherein $R^2$ is $CH_3$ or $CH_2CH_3$ and $R^3$ is H.

4. A process as claimed in any one of claims 1 to 3 wherein $R^4$ is H or $OCH_3$.

5. A process as claimed in any one of claims 1 to 4 wherein n is 2.

6. A process as claimed in claim 1 which comprises reacting N-[2-(N'-4-methanesulphonamidophenethyl-N'-methyl-amino)ethyl]-4-amino-2-methoxybenzamide with methanesulphonyl chloride to give the compound of formula (I) wherein $R^1$ is $CH_3$, $R^2$ is $CH_3$, $R^3$ is H, $R^4$ is $CH_3O$ attached at the 2-position of the phenyl ring and $R^5$ is $CH_3$, the group $-NHSO_2R^5$ being attached at the 4-position of the phenyl ring, and n is 2.

7. A process for preparing a pharmaceutical composition characterised by mixing a compound of the formula (I) as defined in claim 1, or a pharmaceutically acceptable salt thereof, with a pharmaceutically acceptable diluent or carrier.

**Claims for the following Contracting State : GR**

1. A process for preparing a compound having the formula :

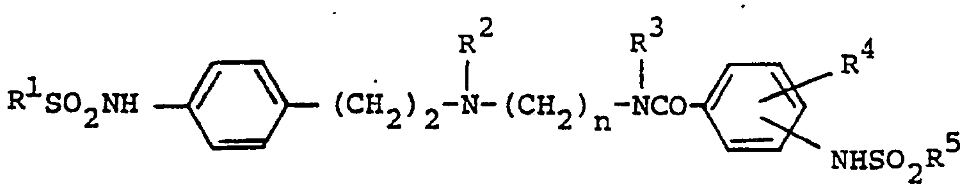

--- (I)

or a pharmaceutically acceptable salt thereof,
wherein $R^1$ and $R^5$ are each independently $C_1$-$C_4$ alkyl,

$R^2$ and $R^3$ are each independently H or $C_1$-$C_4$ alkyl ;

$R^4$ is H or $C_1$-$C_4$ alkoxy,

and n is 2, 3 or 4, which comprises reacting a compound of the formula :

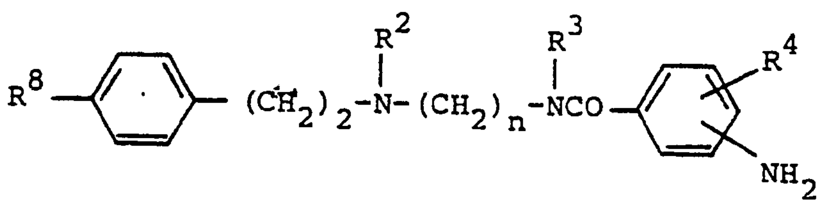

--- (III)

wherein $R^2$, $R^3$, $R^4$ and n are as previously defined and $R^8$ is $NH_2$ or ($C_1$-$C_4$ alkyl)$SO_2NH$ ; with a $C_1$-$C_4$ alkyl sulphonylchloride or bromide, or a sulphonic anhydride of formula $[(C_1\text{-}C_4\ alkyl)SO_2]_2O$.

2. A process as claimed in claim 1 wherein $R^1$ and $R^5$ are both $CH_3$.

3. A process as claimed in claim 1 or claim 2 wherein $R^2$ is $CH_3$ or $CH_2CH_3$ and $R^3$ is H.

4. A process as claimed in any one of claims 1 to 3 wherein $R^4$ is H or $OCH_3$.

5. A process as claimed in any one of claims 1 to 4 wherein n is 2.

6. A process as claimed in claim 1 which comprises reacting N-[2-(N'-4-methanesulphonamidophenethyl-N'-methyl-amino)ethyl]-4-amino-2-methoxybenzamide with methanesulphonyl chloride to give the compound of formula (I) wherein $R^1$ is $CH_3$, $R^2$ is $CH_3$, $R^3$ is H, $R^4$ is $CH_3O$ attached at the 2-position of the phenyl ring and $R^5$ is $CH_3$, the group $-NHSO_2R^5$ being attached at the 4-position of the phenyl ring, and n is 2.

7. A process for preparing a pharmaceutical composition characterised by mixing a compound of the formula (I) as defined in claim 1, or a pharmaceutically acceptable salt thereof, with a pharmaceutically acceptable diluent or carrier.

8. A compound of the formula :

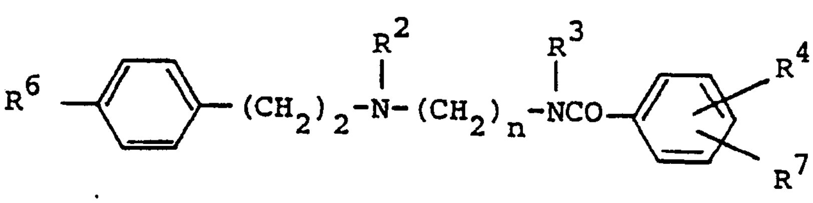

--- (II)

wherein $R^2$, $R^3$, $R^4$ and n are as defined in claim 1 and $R^6$ and $R^7$ are each $NH_2$ or $NO_2$, or $R^6$ is ($C_1$-$C_4$ alkyl)$SO_2NH$ and $R^7$ is $NH_2$ or $NO_2$.

## Ansprüche

### Patentansprüche für die Vertragsstaaten : AT, BE, CH, DE, FR, GB, IT, LI, LU, NL, SE

1. Verbindung der Formel :

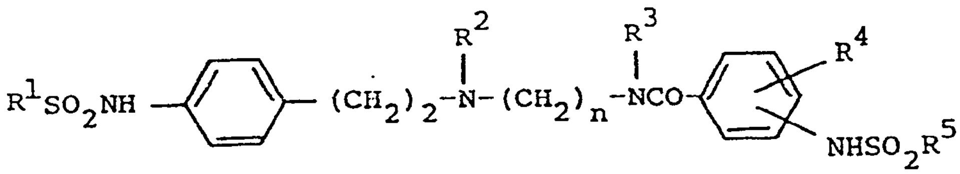

(I)

oder ein pharmazeutisch annehmbares Salz hievon, worin

$R^1$ und $R^5$ jeweils unabhängig $C_1$-$C_4$-Alkyl sind,
$R^2$ und $R^3$ jeweils unabhängig H oder $C_1$-$C_4$-Alkyl bedeuten,
$R^4$ H oder $C_1$-$C_4$-Alkoxy darstellt und
n 2, 3 oder 4 ist.

2. Verbindung nach Anspruch 1, worin $R^1$ und $R^5$ beide $CH_3$ bedeuten.

3. Verbindung nach Anspruch 1 oder 2, worin $R^2$ $CH_3$ oder $CH_2CH_3$ darstellt und $R^3$ die Bedeutung H hat.

4. Verbindung nach einem der Ansprüche 1 bis 3, worin $R^4$ H oder $OCH_3$ darstellt.

5. Verbindung nach einem der Ansprüche 1 bis 4, worin n 2 ist.

6. Verbindung der Formel (I), worin $R^1$ $CH_3$ bedeutet, $R^2$ $CH_3$ ist, $R^3$ die Bedeutung H hat, $R^4$ $CH_3O$ darstellt, das sich in Stellung 2 des Phenylringes befindet, und $R^5$ $CH_3$ bedeutet, wobei sich die Gruppe $-NHSO_2R^5$ in Stellung 4 des Phenylringes befindet, und n 2 ist.

7. Pharmazeutische Zusammensetzung, die eine Verbindung der Formel (I) oder ein pharmazeutisch annehmbares Salz hievon, wie in einem der Ansprüche 1 bis 6 beansprucht, zusammen mit einem pharmazeutisch annehmbaren Verdünnungsmittel oder Träger umfaßt.

8. Verbindung der Formel (I) oder ein pharmazeutisch annehmbares Salz hievon, wie in einem der Ansprüche 1 bis 6 beansprucht, zur Verwendung in der Medizin, insbesondere zur Verwendung als Antiarrhythmiemittel.

9. Verwendung einer Verbindung der Formel (I) oder eines pharmazeutisch annehmbaren Salzes hievon, wie in einem der Ansprüche 1 bis 6 beansprucht, zur Herstellung eines Medikaments zur Verhütung oder Verminderung von Herzarrhythmien.

10. Verbindung der Formel :

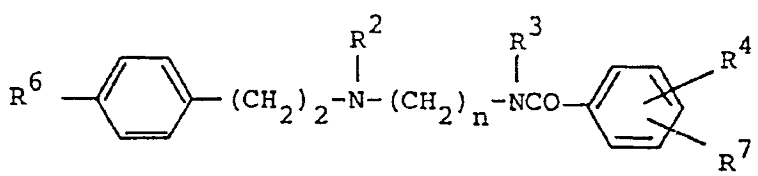

(II),

worin $R^2$, $R^3$, $R^4$ und n wie in Anspruch 1 definiert sind und $R^6$ und $R^7$ jeweils $NH_2$ oder $NO_2$ bedeuten oder $R^6$ ($C_1$-$C_4$-Alkyl)$SO_2NH$ ist und $R^7$ $NH_2$ oder $NO_2$ darstellt.

### Patentansprüche für den Vertragsstaat : ES

1. Verfahren zum Herstellen einer Verbindung der Formel :

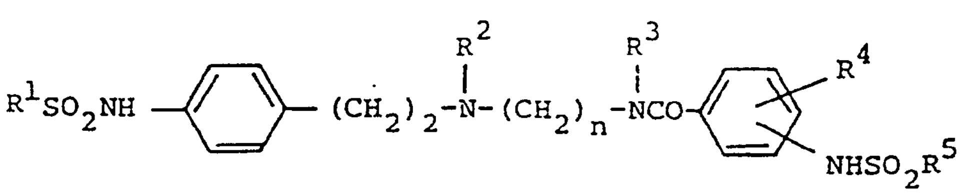

(I)

oder eines pharmazeutisch annehmbaren Salzes hievon, worin

$R^1$ und $R^5$ jeweils unabhängig $C_1$-$C_4$-Alkyl sind,

$R^2$ und $R^3$ jeweils unabhängig H oder $C_1$-$C_4$-Alkyl bedeuten,

$R^4$ H oder $C_1$-$C_4$-Alkoxy darstellt und

n 2, 3 oder 4 ist.

das das Umsetzen einer Verbindung der Formel :

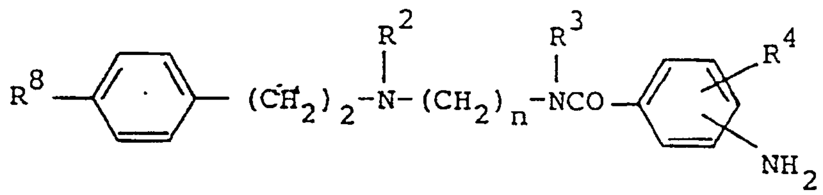

(III),

worin $R^2$, $R^3$, $R^4$ und n wie oben definiert sind und $R^8$ $NH_2$ oder ($C_1$-$C_4$-Alkyl)$SO_2NH$ bedeutet, mit einem $C_1$-$C_4$-Alkylsulfonylchlorid oder -bromid oder einem Sulfonsäureanhydrid der Formel [($C_1$-$C_4$-Alkyl)$SO_2$]$_2$O umfaßt.

2. Verfahren nach Anspruch 1, worin $R^1$ und $R^5$ beide $CH^3$ bedeuten.

3. Verfahren nach Anspruch 1 oder 2, worin $R^2$ $CH_3$ oder $CH_2CH_3$ darstellt und $R^3$ die Bedeutung H hat.

4. Verfahren nach einem der Ansprüche 1 bis 3, worin $R^4$ H oder $OCH_3$ darstellt.

5. Verfahren nach einem der Ansprüche 1 bis 4, worin n 2 ist.

6. Verfahren nach Anspruch 1, das das Umsetzen von N-[2-(N'-4-Methansulfonamidophenäthyl-N'-methylamino)-äthyl]-4-amino-2-methoxybenzamid mit Methansulfonylchlorid zu der Verbindung der Formel (I), worin $R^1$ $CH_3$ bedeutet, $R^2$ CH3 ist, $R^3$ die Bedeutung H hat, $R^4$ $CH_3O$ darstellt, das sich in Stellung 2 des Phenylringes be findet, und $R^5$ $CH_3$ bedeutet, wobei sich die Gruppe $-NHSO_2R^5$ in Stellung 4 des Phenylringes befindet, und n 2 ist, umfaßt.

7. Verfahren zum Herstellen einer pharmazeutischen Zusammensetzung, dadurch gekennzeichnet, daß man eine eine Verbindung der Formel (I), wie in Anspruch 1 definiert, oder ein pharmazeutisch annehmbares Salz hievon mit einem pharmazeutisch annehmbaren Verdünnungsmittel oder Träger mischt.

**Patentansprüche für den Vertragsstaat : GR**

1. Verfahren zum Herstellen einer Verbindung der Formel :

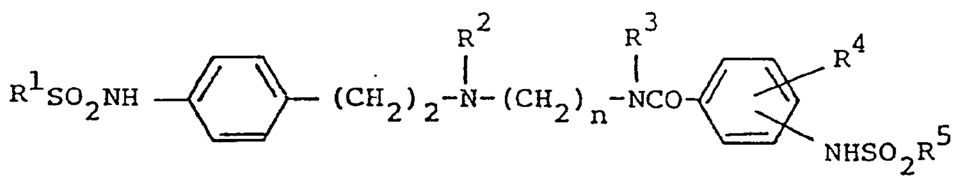

(I)

oder eines pharmazeutisch annehmbaren Salzes hievon, worin

$R^1$ und $R^5$ jeweils unabhängig $C_1$-$C_4$-Alkyl sind,

$R^2$ und $R^3$ jeweils unabhängig H oder $C_1$-$C_4$-Alkyl bedeuten,

$R^4$ H oder $C_1$-$C_4$-Alkoxy darstellt und

n 2, 3 oder 4 ist.

das das Umsetzen einer Verbindung der Formel :

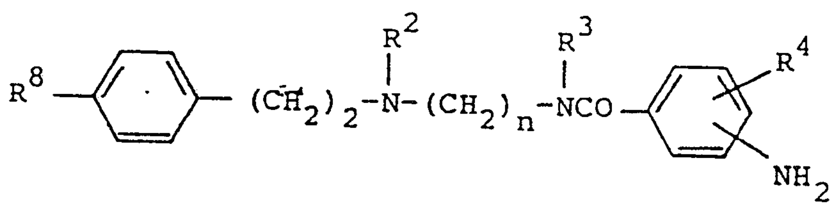

(III),

worin $R^2$, $R^3$, $R^4$ und n wie oben definiert sind und $R^8$ $NH_2$ oder ($C_1$-$C_4$-Alkyl)$SO_2NH$ bedeutet, mit einem $C_1$-$C_4$-Alkylsulfonylchlorid oder -bromid oder einem Sulfonsäureanhydrid der Formel [($C_1$-$C_4$-Alkyl)$SO_2$]20 umfaßt.

2. Verfahren nach Anspruch 1, worin $R^1$ und $R^5$ beide $CH^3$ bedeuten.

3. Verfahren nach Anspruch 1 oder 2, worin $R^2$ $CH_3$ oder $CH_2CH_3$ darstellt und $R^3$ die Bedeutung H hat.

4. Verfahren nach einem der Ansprüche 1 bis 3, worin $R^4$ H oder $OCH_3$ darstellt.

5. Verfahren nach einem der Ansprüche 1 bis 4, worin n 2 ist.

6. Verfahren nach Anspruch 1, das das Umsetzen von N-[2-(N'-4-Methansulfonamidophenäthyl-N'-methylamino)-äthyl]-4-amino-2-methoxybenzamid mit Methansulfonylchlorid zu der Verbindung der Formel (I), worin $R^1$ $CH_3$ bedeutet, $R^2$ $CH_3$ ist, $R^3$ die Bedeutung H hat, $R^4$ $CH_3O$ darstellt, das sich in Stellung 2 des Phenylringes be findet, und $R^5$ $CH_3$ bedeutet, wobei sich die Gruppe $-NHSO_2R^5$ in Stellung 4 des Phenylringes befindet, und n 2 ist, umfaßt.

7. Verfahren zum Herstellen einer pharmazeutischen Zusammensetzung, dadurch gekennzeichnet, daß man eine eine Verbindung der Formel (I), wie in Anspruch 1 definiert, oder ein pharmazeutisch annehmbares Salz hievon mit einem pharmazeutisch annehmbaren Verdünnungsmittel oder Träger mischt.

8. Verbindung der Formel :

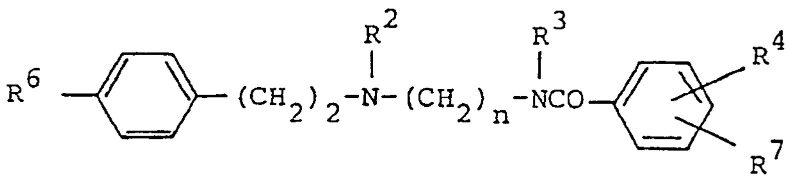

(II),

worin $R^2$, $R^3$, $R^4$ und n wie in Anspruch 1 definiert sind und $R^6$ und $R^7$ jeweils $NH_2$ oder $NO_2$ bedeuten oder $R^6$ $(C_1\text{-}C_4\text{-Alkyl})SO_2NH$ ist und $R^r$ $NH_2$ oder $NO_2$ darstellt.

## Revendications

### Revendications pour les Etats contractants : AT, BE, CH, DE, FR, GB, IT, LI, LU, NL, SE

1. Composé de formule :

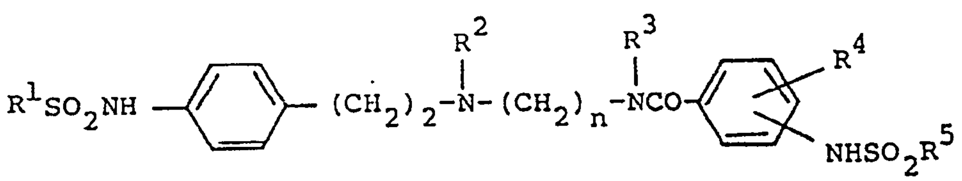

---(I)

ou sel pharmaceutiquement acceptable d'un tel composé, dans laquelle

$R^1$ et $R^5$, identiques ou différents, représentent un groupe alkyle en $C_1$-$C_4$ ;

$R^2$ et $R^3$, identiques ou différents, représentent H ou un groupe alkyle en $C_1$-$C_4$ ;

$R^4$ représente H ou un groupe alcoxy en $C_1$-$C_4$ ; et

n représente 2, 3 ou 4.

2. Composé selon la revendication 1, dans lequel $R^1$ et $R^5$ représentent tous deux $CH_3$.

3. Composé selon la revendication 1 ou 2, dans lequel $R^2$ représente $CH_3$ ou $CH_2CH_3$ et $R^3$ représente H.

4. Composé selon l'une quelconque des revendications 1 à 3, dans lequel $R^4$ représente H ou $OCH_3$.

5. Composé selon l'une quelconque des revendications 1 à 4, dans lequel n représente 2.

6. Composé de formule (I), dans lequel $R^1$ représente $CH_3$, $R^2$ représente CH, $R^3$ représente H, $R^4$ représente $CH_3O$ fixé en position-2 sur le noyau phényle, et $R^5$ représente $CH_3$, le groupe $-NHSO_2R^5$ étant fixé en position-4 sur le noyau phényle, et n représente 2.

7. Composition pharmaceutique comprenant un composé de formule (I) ou un sel pharmaceutiquement acceptable d'un tel composé tel que revendiqué selon l'une quelconque des revendications 1 à 6 avec un diluant ou un véhicule pharmaceutiquement acceptable.

8. Composé de formule (I) ou sel pharmaceutiquement acceptable d'un tel composé, selon l'une quelconque des revendications 1 à 6, destiné à l'utilisation en médecine, en particulier comme agent anti-arythmique.

9. Utilisation du composé de formule (I) ou d'un sel pharmaceutiquement acceptable d'un tel composé selon l'une quelconque des revendications 1 à 6, pour la fabrication d'un médicament en vue de la prévention ou de la réduction des arythmies cardiaques.

10. Composé de formule :

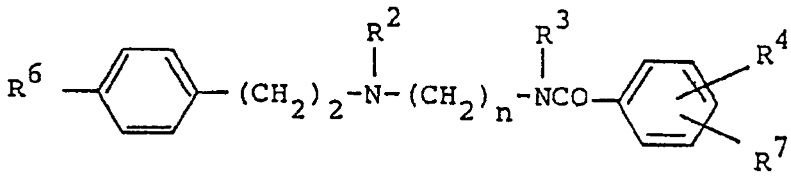

---(II)

dans laquelle $R^2$, $R^3$, $R^4$ et n sont tels que définis dans la revendication 1, et $R^6$ et $R^7$ représentent chacun $NH_2$ ou $NO_2$, ou $R^6$ est un groupe (alkyle en $C_1$ -$C_4$)$SO_2$ NH et $R^7$ représente $NH_2$ ou $NO_2$.

**Revendications pour l'Etat contractant : ES**

1. Procédé de préparation d'un composé de formule :

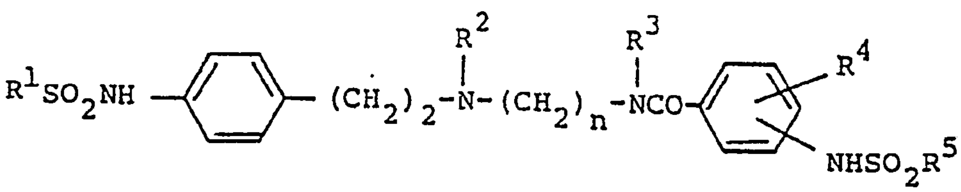

---(I)

ou d'un sel pharmaceutiquement acceptable d'un tel composé, dans laquelle :

$R^1$ et $R^5$, identiques ou différents, représentent un groupe alkyle en $C_1$-$C_4$ ;
$R^2$ et $R^3$, identiques ou différents, représentent h ou alkyle en $C_1$-$C_4$ ;
$R^4$ représente H ou un groupe alcoxy en $C_1$-$C_4$ ; et
n représente 2, 3 ou 4,

qui consiste à faire réagir un composé de formule :

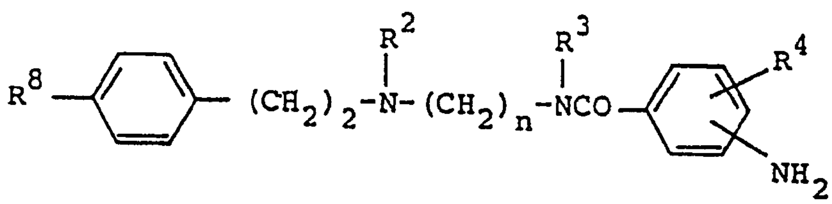

---(III)

dans laquelle $R^2$, $R^3$, $R^4$ et n sont tels que précédemment définis, et $R^8$ représente $NH_2$ ou un groupe (alkyle en $C_1$ -$C_4$)-$SO_2$ NH ; avec un sulfonyl -chlorure ou -bromure d'alkyle en $C_1$-$C_4$, ou un anhydride sulfonique de formule ((alkyle en $C_1$-$C_4$)$SO_2$)$_2$ O.

2. Procédé selon la revendication 1, dans lequel $R^1$ et $R^5$ représentent tous deux $CH_3$.

3. Procédé selon la revendication 1 ou 2, dans lequel $R^2$ représente $CH_3$ ou $CH_2CH_3$, et $R^3$ représente H.

4. Procédé selon l'une quelconque des revendications 1 à 3, dans lequel $R^4$ représente H ou $OCH_3$.

5. Procédé selon l'une quelconque des revendications 1 à 4, dans lequel n représente 2.

6. Procédé selon la revendication 1, qui consiste à faire réagir le N-(2-(N'-4-méthanesulfonamidophénéthyl-N'-méthylamino)éthyl)-4-amino-2-méthoxybenzamide avec le chlorure de méthanesulfonyle pour donner le composé de formule (I) dans lequel $R^1$ représente $CH_3$, $R^2$ représente $CH_3$, $R^3$ représente H, $R^4$ représente $CH_3O$ fixé en position-2 sur le noyau phényle et $R^5$ représente $CH_3$, le groupe -$NHSO_2R^5$ étant fixé en position-4 sur le noyau phényle, et n représente 2.

7. Procédé de préparation d'une composition pharmaceutique, caractérisé en ce qu'il consiste à mélanger un composé de formule (I) tel que défini dans la revendication 1, ou un sel pharmaceutiquement acceptable d'un tel composé, avec un diluant ou un véhicule pharmaceutiquement acceptable.

**Revendications pour l'Etat contractant : GR**

1. Procédé de préparation d'un composé de formule :

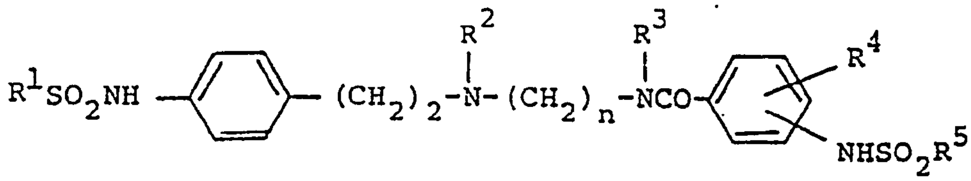

----(I)

ou d'un sel pharmaceutiquement acceptable d'un tel composé, dans laquelle :

$R^1$ et $R^5$, identiques ou différents, représentent un groupe alkyle en $C_1$-$C_4$ ;
$R^2$ et $R^3$, identiques ou différents, représentent H ou alkyle en $C_1$-$C_4$ ;
$R^4$ représente H ou un groupe alcoxy en $C_1$-$C_4$ ; et
n représente 2, 3 ou 4,

qui consiste à faire réagir un composé de formule :

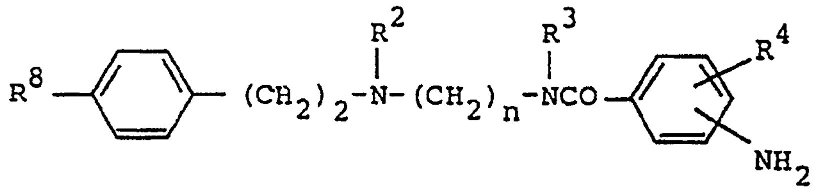

---(III)

dans laquelle $R^2$, $R^3$, $R^4$ et n sont tels que précédemment définis, et $R^8$ représente $NH_2$ ou un groupe (allyle en $C_1$ -$C_4$)-$SO_2$ NH ; avec un sulfonyl -chlorure ou -bromure d'allyle en $C_1$-$C_4$, ou un anhydride sulfonique de formule ((allyle en $C_1$-$C_4$)$SO_2$)$_2$ O.

2. Procédé selon la revendication 1, dans lequel $R^1$ et $R^5$ représentent tous deux $CH_3$.

3. Procédé selon la revendication 1 ou 2, dans lequel $R^2$ représente $CH_3$ ou $CH_2CH_3$, et $R^3$ représente H.

4. Procédé selon l'une quelconque des revendications 1 à 3, dans lequel $R^4$ représente H ou $OCH_3$.

5. Procédé selon l'une quelconque des revendications 1 à 4, dans lequel n représente 2.

6. Procédé selon la revendication 1, qui consiste à faire réagir le N-(2-(N'-4-méthanesulfonamidophénéthyl-N'-méthylamino)éthyl)-4-amino-2-méthoxybenzamide avec le chlorure de méthanesulfonyle pour donner le composé de formule (I) dans lequel $R^1$ représente $CH_3$, $R^2$ représente $CH_3$, $R^3$ représente H, $R^4$ représente $CH_3O$ fixé en position-2 sur le noyau phényle et $R^5$ représente $CH_3$, le groupe -$NHSO_2R^5$ étant fixé en position-4 sur le noyau phényle, et n représente 2.

7. Procédé de préparation d'une composition pharmaceutique, caractérisé en ce qu'il consiste à mélanger un composé de formule (I) tel que défini dans la revendication 1, ou un sel pharmaceutiquement acceptable d'un tel composé, avec un diluant ou un véhicule pharmaceutiquement acceptable.

8. Composé de formule :

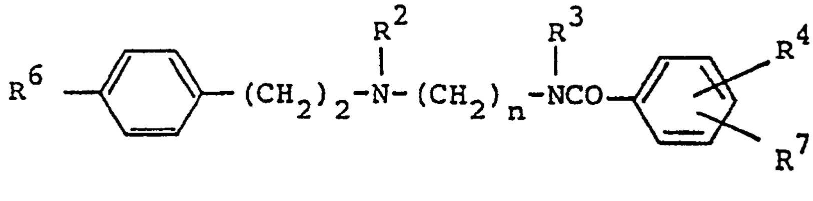

(II)

dans lequel $R^2$, $R^3$, $R^4$ et n sont tels que définis dans la revendication 1, et $R^6$ et $R^7$ représentent chacun $NH_2$ ou $NO_2$, ou $R^6$ représente (alkyle en $C_1$ -$C_4$)$SO_2$ NH et $R^7$ représente $NH_2$ ou $NO_2$.